(19) Europäisches Patentamt
European Patent Office
Office européen des brevets

(11) **EP 1 471 952 B1**

(12) **EUROPEAN PATENT SPECIFICATION**

(45) Date of publication and mention
of the grant of the patent:
**21.07.2010 Bulletin 2010/29**

(51) Int Cl.:
***A61L 27/34*** *(2006.01)*     ***A61L 27/56*** *(2006.01)*

(21) Application number: **03713372.5**

(86) International application number:
**PCT/US2003/003481**

(22) Date of filing: **05.02.2003**

(87) International publication number:
**WO 2003/066118 (14.08.2003 Gazette 2003/33)**

(54) **COATED VASCULAR PROSTHESIS AND METHODS OF MANUFACTURE AND USE.**

BESCHICHTETE GEFÄSSPROTHESEN, VERFAHREN ZU DEREN HERSTELLUNG UND VERWENDUNG DERSELBEN

PROTHESE VASCULAIRE ENROBEE, ET PROCEDES DE FABRICATION ET D'UTILISATION ASSOCIES

(84) Designated Contracting States:
**DE ES FR GB IT**

(30) Priority: **05.02.2002 US 354727 P**

(43) Date of publication of application:
**03.11.2004 Bulletin 2004/45**

(73) Proprietor: **Thoratec Corporation
Pleasanton, CA 94588 (US)**

(72) Inventors:
  • **JAYARAMAN, Ramesh, B.
Fremont, CA 94539 (US)**

  • **BRIANA, Stephen, G.
Danville, CA 94525 (US)**

(74) Representative: **Lawrence, John et al
Barker Brettell LLP
100 Hagley Road
Edgbaston
Birmingham B16 8QQ (GB)**

(56) References cited:
  **WO-A-91/05522**     **US-A- 4 604 762**
  **US-A- 5 298 276**

**Description**

BACKGROUND OF THE INVENTION

Field of the Invention

The present invention relates generally to vascular grafts and other vascular prostheses. More particularly, the present invention relates to methods for making a porous vascular prosthesis comprising a fabric substrate and two distinctive polymer coatings. The prostheses of the invention provide improved resistance to blood permeability without undue kinking. The prostheses of the invention are useful in medical applications, including use as the outflow graft of a ventricular assist device.

Description of Related Art

Congestive heart failure is reaching epidemic proportions in the United States. Approximately five million Americans have congestive heart failure, and about 400,000 new cases are diagnosed each year. In congestive heart failure, the patient's own heart fails to function at a level sufficient to maintain adequate blood flow to the vital organs of the body.
One approach for the treatment of congestive heart failure involves implanting a ventricular assist device (VAD). There are several types of ventricular assist devices. Some ventricular assist devices are only intended to perform the function of the left ventricle, while other devices can perform the function of either the left or right ventricle. Ventricular assist devices are pumps that receive blood from the left or right ventricle, and discharge blood to either the ascending aorta in the case of the left ventricle, or the pulmonary artery in the case of the right ventricle. The blood conducting inlet to the device is typically a fairly rigid tube, which defines a distance of the pump from the ventricle, in order to provide for correct anatomical placement of the pump with respect to the ventricle. In contrast, the blood conducting outlet from the device is typically a flexible tube that can accommodate bending to the path necessary to reach the ascending aorta or the pulmonary artery. Because of differences in individual patient anatomy, ideally, the flexible tube can be cut to the appropriate length for each patient. One flexible material that has been used for the outlet (and in some cases, the inlet) of ventricular assist devices is a polyester vascular graft.
Grafts incorporating polyester fabric have been used for many years as replacements for native blood vessels in humans, and as conduits leading to or from ventricular assist devices. However, because these grafts are constructed from polyester fibers, there are spaces or pores between the fibers of variable size, depending on whether the fibers are knitted or woven and how tightly the fibers are packed. These spaces between the polyester fibers may allow blood to flow through the graft wall under some conditions. This situation has been addressed in the past by impregnation of the polyester graft material with either gelatin or collagen. This results in a blood impermeable graft at the time of implantation, but one which has a blood contacting surface that is no longer pure polyester. Over time, the collagen or gelatin resorbs, and the underlying polyester graft is exposed to the bloodstream and surrounding tissues. Since this process takes place over a reasonably long time, a blood impermeable native surface typically forms over the polyester graft as the collagen or gelatin resorbs. On the external surface of the graft, the resorption of the collagen or gelatin results in a polyester surface sufficiently porous to allow tissue to ingrow and attach. While this ingrowth may not be detrimental to the patient while the device is in place, many patients with a ventricular assist device will undergo subsequent heart transplant surgery. Before transplantation, the ventricular assist device must be surgically removed. If there is tissue ingrowth into the exterior surface of the graft, the tissue must be dissected from the graft prior to removal of the device. This requires additional surgical time and has the potential to cause bleeding complications. It is therefore desirable that the exterior of the graft have a surface that does not allow for or inhibits ingrowth and attachment of tissue. US 5,298,276 discloses an artificial blood vessel comprising a knitted or woven crimped tube and one or more coating layers of polysiloxane or polyurethane. WO 91/05522 discloses a vascular prosthesis having a polymeric coating layer. US 4,604,762 discloses an arterial graft prosthesis comprising two layers of elastomeric material, the inner layer being porous.
Therefore, a need has arisen for a vascular prosthesis that can be used as part of a VAD that provides a strong, compliant, kink resistant barrier, while at the same time providing long and short term resistance against leakage of blood through the pores of the fabric.

SUMMARY OF THE INVENTION

This object can be achieved by the prosthesis and the methods according to the independent claims. Further enhancements are characterized in the dependent claims. The present invention is directed to vascular prostheses made of fabric substrates having two distinctive porous coatings made of biocompatible polymers, such as Thoralon® polymer. Preferred embodiments provide kink resistant, yet blood impermeable barriers. The exterior coatings of preferred embodiments of the invention discourage tissue ingrowth on the exterior surface of the prostheses. Further, because the inner or blood interface surface of the fabric substrates are left uncoated, the blood compatibility of the base graft material in these embodiments is maintained.

Prosthesis according to the invention are particularly useful as outflow grafts or cannulas for ventricular assist devices and for the repair of vascular defects in large vessels, such as the ascending and abdominal aorta. However, vascular prostheses coated according to the invention are not limited to these applications, and may be broadly used in a variety of applications, including vascular grafts (including stent grafts) and vascular patches for any area of the body. Coated grafts according to preferred embodiments of the present invention are biocompatible, biostable, compliant, strong and resistant to kinking.

For example, one embodiment of the invention is directed to a method for making a vascular prosthesis comprising the steps of : providing a crimped substrate, the substrate comprising a woven or knitted polymer fabric, the substrate having a first surface and a second surface opposite the first surface, the second surface being a blood interface surface; coating the first surface with a first solution, the first solution comprising a first polymer and a particulate in a first solvent; allowing the first solution to dry, thereby forming a first coat on the substrate; coating the first coat with a second solution, the second solution comprising a second polymer in a second solvent; and immersing the coated substrate in a third solvent to precipitate the second polymer from solution, thereby forming a porous second coat.

Another embodiment is directed to a method for sealing the pores of a fabric graft comprising the steps of: applying a first solution to a first surface of the graft, the first solution comprising a polyurethane and a salt in a first solvent; allowing the first solution to dry, thereby forming a first coat on the graft; applying a second solution to the coated first surface, the second solution comprising a second polyurethane in a second solvent; and immersing the coated graft in water to precipitate the second polyurethane from solution, thereby forming a porous second coat, and dissolving the salt in the first coat to form pores in the first coat.

Still another embodiment is directed to a vascular prosthesis comprising: a substrate comprising a crimped woven or knitted polymer fabric, the substrate having a first surface and a second surface opposite the first surface, the second surface being a blood interface surface; a first porous coating disposed on the first surface, the first porous coating comprising a first polymer; and a second porous coating disposed on the first porous coating, the second porous coating comprising a second polymer. The first coating does not penetrate to the second surface of the substrate.

Another embodiment is directed to a vascular prosthesis comprising: a first layer comprising a crimped woven or knitted polymer fabric having a first surface; a second layer comprising a first porous coating disposed on the first surface, the first porous coating comprising a first polymer; and a third layer comprising a second porous coating disposed on the first porous coating, the second porous coating comprising a second polymer. The second layer is disposed between the first and third layers. The first porous coating does not penetrate to the second surface of the first layer.

Other objects, features, and advantages will be apparent to those skilled in the art in view of the following description of the preferred embodiments and the accompanying drawings. In the drawings, like reference numerals refer to like elements or features.

## BRIEF DESCRIPTION OF THE DRAWINGS

The present invention may be more readily understood with reference to the following drawing in which:

**Fig. 1** is an electron micrograph of a coated vascular prosthesis according to one embodiment of the invention.

**Fig. 2** is a perspective view of a portion of a VAD.

**Fig. 3** is a cross-sectional view of the outflow cannula of the VAD of **Fig. 2**

**Fig. 4** is an enlarged view of the outflow graft portion of the outflow cannula of **Fig. 3.**

**Fig. 5** depicts a template used to measure kink diameter.

## DESCRIPTION OF PREFERRED EMBODIMENTS

The present invention is directed to vascular prostheses made of porous fabrics, such as woven polyester, coated with two distinctive porous coatings of Thoralon® polymer or another suitable polymer, to prevent leakage of blood through the pores of the fabric. As used herein, "vascular prostheses" broadly includes, but is not limited to, vascular grafts, stent grafts, vascular patches, inflow and outflow cannulas for heart assist devices and components of native or man-made hearts and heart assist devices. Specifically, preferred embodiments of the present invention use biocompatible polyurethanes, such as Thoralon® polymer, to create distinctive porous coatings for textile or fabric grafts. Coated grafts according to the invention have improved impermeability and are thus less prone to allow leakage of fluids, such as blood, serum or water, through the fabric of the graft, both long and short term.

Because polyurethanes have very low water permeability, they may effectively seal a textile. Furthermore, polyurethanes, such as Thoralon® polymer, possess a number of desirable properties such as biostability, compliance, biocompatibility, blood compatibility and strength, which are important in many vascular applications. As such, polyurethane coated textiles according to preferred embodiments of the invention provide improved biocompatibility, as well as strong and compliant reinforcement or replacement of the diseased area.

Further, by incorporating a novel combination of porous layers, preferred embodiments of the invention also solve the problems of kinking and tissue ingrowth on the exterior surface of the graft which may occur with other coatings.

The use of salt particles in polyurethane solutions to create pores in polyurethane has previously been described. However, it has surprisingly been discovered that the *amount* of salt may be varied to accomplish two other unexpected purposes: reduction of kinking and prevention of excess penetration of the coating materials into the graft.

As shown in the Examples, when a coating of evaporated or precipitated Thoralon® polymer solution (without salt) was applied directly over a fabric graft, the resulting graft had an increased kink diameter as compared to the uncoated graft. However, by first coating the graft with a solution containing an appropriate amount of salt (Example 6), and then overcoating with a second Thoralon® polymer solution (without salt) and precipitating, it was discovered that more of the original kink resistance of the base graft is maintained. The salt containing solution acts as a masking layer over the graft, such that the second Thoralon® polymer solution does not permeate into the fabric.

The amount of salt particles also controls the permeation of the salt containing solution into the fabric and thus the adherence of the coating to the fabric. This can be seen by comparing Examples 5-8. In the case of no or low salt content, kink diameter increases (i.e., the graft kinks more easily) due to excess permeation into the fabric. With increasing salt content, permeation is reduced and the kink diameter is reduced. However, if the salt content is too high, the first coating does not penetrate enough into the fabric to provide adherence after pressurizing the graft. Thus, by providing the appropriate level of salt or another particulate, adequate adherence is obtained, kink resistance is optimized and the coatings do not penetrate to the blood contacting surface of the graft. The latter feature is particularly useful in grafts used as VAD outflow cannulas, where it may be desirable to keep the blood contacting surface of the graft free of the polymer coatings.

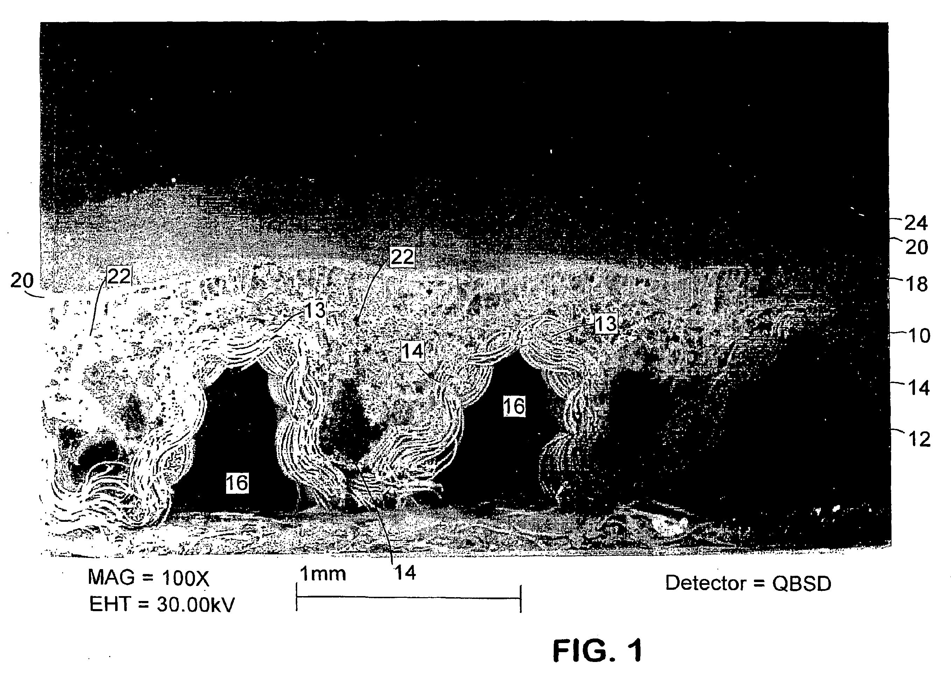

Applying these findings, it has been discovered that a blood impermeable, kink resistant vascular prosthesis or graft may be prepared by coating the outer surface of a graft with two distinctive porous polymer coatings or layers. An electron micrograph showing a coated graft prepared according to a preferred embodiment of the invention is shown in Figure 1. As shown in Figure 1, graft 10 comprises a substrate or supporting component 12. Substrate 12 has a first surface 14, and a second surface 16. Preferably, second surface 16 is the blood interface surface of the graft. A first coat or layer 18 is disposed on the first surface of the graft. A second coat or layer 20 is disposed over the first coat.

Substrate 12 is preferably a crimped polyester woven vascular graft, such as a Cooley Low Porosity ™ graft available from Boston Scientific/Medi-Tech (Natick, MA). This graft has an accordion like structure with peaks 13. Although the preferred embodiment shown in **Fig. 1** uses woven polyester fabric, other fabrics may be used without departing from the invention. Such fabrics include, but are not limited to, knitted or woven Dacron® (polyethylene terephthalate or PET), Teflon® material (expanded polytetrafluoroethylene or ePTFE) and PTFE (polytetrafluoroethylene).

As shown in **Fig.1,** first coat or layer 18 is disposed on first surface 14 of substrate 12. First coat 18 preferably made from a first solution comprising a polyurethane, such as Thoralon® polymer, and a particulate, such as a salt, in a suitable first solvent, such as N,N dimethyl acetamide (referred to herein as DMAC or dimethyl acetamide). The particulate is insoluble in the first solvent.

As discussed below, the first solution is not limited to Thoralon® polymer and salt in DMAC. The first solution may alternately comprise one or more of a variety of different polymers in a variety of different solvents. Further, a variety of different particulates or pore forming agents may be used.

As will be clear to those of skill in the art, the precise amounts of the first polymer, first solvent and particulate will vary, depending on the particular ingredients used. The particulate is added in the amount determined necessary to achieve the desired level of penetration into the substrate. For example, in a preferred embodiment, in which the first polymer is Thoralon® polymer, the solvent is DMAC and the particulate is NaCl, the amount of the first polymer is preferably about 7.3% by weight of the final solution, the amount of first solvent is preferably about 50.6% by weight of the final solution, and the amount of salt is preferably about 42.1% by weight of the final solution.

After allowing first coat 18 to oven dry, a second coat or layer 20 is applied over first coat 18. Second coat 20 is preferably made from a second solution comprising a polyurethane, such as Thoralon® polymer, in a suitable solvent, such as DMAC. However, as discussed below, like the first solution, a variety of different polymers and solvents may be used in the second solution.

The precise amount of second polymer and second solvent in the second solution will vary, depending on the particular ingredients used. In a preferred embodiment, the amount of the second polymer preferably ranges from 3% to 20%, more preferably from 5% to 15% and most preferably about 8% by weight of the final solution. In this embodiment, the amount of the second solvent preferably ranges from 80% to 97%, more preferably from 85% to 95% and most preferably about 92% by weight of the final solution.

After application of the second coat, the coated graft is immersed in water or another suitable solvent to precipitate the Thoralon® polymer or other polymer from the second solution. Precipitation causes pores 24 to form in the second coat 20, thereby forming a porous surface layer on the graft. Preferably, the pores 24 in the second coat or surface layer 20 are less than about 10 microns in size. The second coat 20 preferably has an average porosity of between about 50% and about 90% by volume. The water also causes the salt in the first solution to dissolve, forming pores 22 in first coat 18. Pores 22 in first coat 18 preferably average between about 5 and about 100 microns in size. First coat 18 preferably has an average porosity of between about 50% and about 90% by volume. For example, in one embodiment, the first porous coating may have an average pore size of about 15 microns and an average porosity of about 75%.

As noted, it has been discovered that varying the amount of salt present in the first solution determines the level of penetration of the first polymer solution into the fabric. The inclusion of an appropriate amount of salt in the first solution controls penetration and inhibits the "wicking" of the solution into the fabric. For example, a first solution containing 7.3% by weight Thoralon® polymer, 42.1% by weight NaCl, and 50.6% by weight DMAC, with a viscosity of about 6000 centipoise does not wick into the graft fabric so as to negatively impact the kink resistance. In contrast, a solution of equivalent viscosity containing only Thoralon® polymer and DMAC wicks into the fabric, reducing the kink resistance and potentially traveling all the way to the inside surface.

By including an appropriate amount of salt in the first solution, the first coat 18 does not penetrate through to or coat the second surface 16 of the graft, thus preserving the original blood contacting surface. As a result, the crimped woven polyester inner surface, which is known to be blood compatible, is maintained in its original, uncoated state. In addition, the first layer also acts as a shield to prevent the second solution from penetrating through to the inside of the graft. As a result, neither solution penetrates to the second surface. As such, the blood contacting surface of the graft is intentionally not affected by the coating process.

In addition to providing a blood impermeable barrier, the porous coats of the invention may provide enhanced fluid transport to any biological layer formed on the inside or second surface of the graft. The porous layers are also more flexible than a solid coating, thus providing greater kink resistance. For example, as shown in Example 6 below, a double coated graft prepared according to the invention had kink resistance nearly equivalent to the uncoated graft. At the same time, the double coated graft did not allow blood to pass through the walls and had a water permeability of 0.33 ml/min/cm$^2$ at 120 mm Hg. In addition, preferred coatings of the invention adhere to the graft, seal the pore openings, and maintain their mechanical function *in vivo* for a period of years.

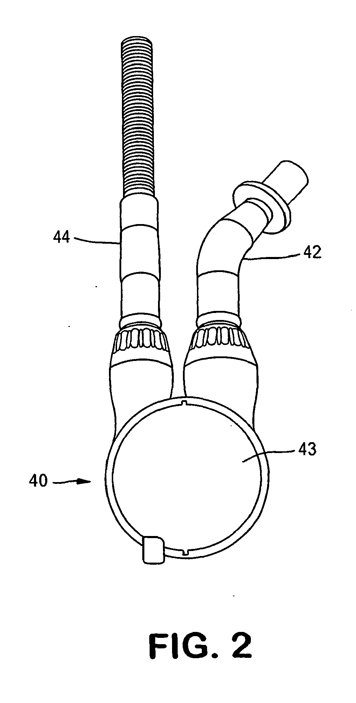

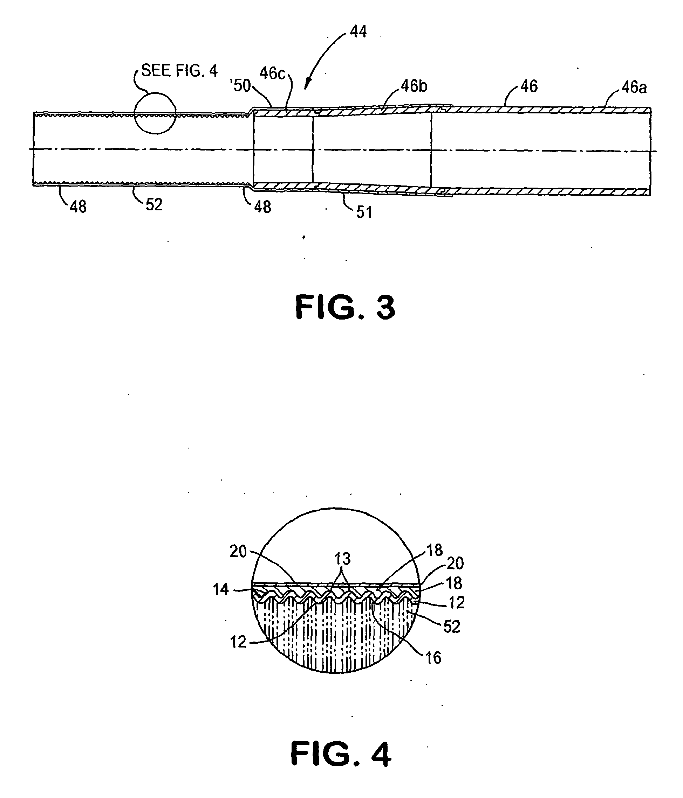

Because of these features, the invention is particularly useful as the outflow cannula of a ventricular assist device. **Fig. 2** shows one example of an implantable portion of a VAD device useful in the practice of the invention. Specifically, VAD device 40 comprises an inflow cannula 42, a pump 43 and an outflow cannula 44. Outflow cannula is adapted for attachment, for example, to a patient's ascending aorta. **Fig. 3** shows a cross section of outflow cannula 44. Specifically, outflow cannula 44 comprises a connection region 46 and an outlet tube portion 48. Connection region 46 preferably comprises first segment 46a, second segment 46b and third segment 46c, all of which segments are preferably made of a polyurethane, such as solid Thoralon® polymer. Second segment 46b may contain wire reinforcement within the walls of the segment. Outlet tube portion 48 comprises an attachment portion 50, which is designed to fit over the outer surface of third segment 46c of connection region 46. Attachment portion 50 preferably comprises uncrimped woven polyester coated according to the invention. Polyester velour 51 may be provided over the outer surface of second segment 46b and over part of first segment 46a to provide a surface for tissue to ingrow. Outlet tube portion 48 further comprises an outflow graft portion 52 which may be cut as desired and is designed for anastomosis directly to the ascending aorta of the patient.

A cross section of outflow graft portion 52 according to a preferred embodiment of the invention is shown in **Fig. 4.** As shown in **Fig. 4,** outflow graft portion 52 comprises an accordion shaped substrate 12, having a first coat 18 and a second coat 20, as previously described.

Accordingly, one embodiment of the invention is directed to a method for making a vascular prosthesis comprising the steps of: providing a crimped substrate, the substrate comprising a woven or knitted polymer fabric, the substrate having a first surface and a second surface opposite the first surface, the second surface being a blood interface surface; coating the first surface with a first solution, the first solution comprising a first polymer and a particulate in a first solvent; allowing the first solution to dry, thereby forming a first coat on the substrate; coating the first coat with a second solution, the second solution comprising a second polymer in a second solvent; and immersing the coated substrate in a third solvent to precipitate the second polymer (which is insoluble in the third solvent) from solution, thereby forming a porous second coat. Preferably, the particulate is soluble in the third solvent and the step of immersing in the third solvent dissolves the particulate in the first coat, thereby causing pores to form in the first coat. For instance, when the third solvent is water, the particulate is preferably a water soluble particulate. Preferably, the particulate in the first solution is present in an amount that inhibits wicking of the first solution into the fabric such that the first solution does not penetrate to the second surface of the fabric.

Preferably, the resulting prosthesis has a kink resistance nearly equivalent to the uncoated fabric. In a preferred embodiment, the prosthesis has a kink diameter of about 12.7 mm (0.5 inches) or less. Preferably, the prosthesis is impermeable to blood, and has a water permeability of less than about 10, more preferably, less than about 5, and most preferably, less than about 0.5 ml/min/cm$^2$ at 120 mm Hg

The substrate may be any suitable fabric. Useful fabrics include, but are not limited to, woven or knitted polyester, Dacron® material, Teflon® material, and PTFE. Preferably, the fabric is crimped to reduce kinking. Most preferably, the fabric is woven crimped polyester. The substrate may assume any desired shape or configuration, including a tube or cylinder, bifurcated Y-shaped cylinder or flat sheet. As such, the prostheses of the invention may be used in a wide variety of vascular applications, including, but not limited to, any type of vascular graft or patch, or as a component of a natural or man-made heart or a heart assist device. In a preferred embodiment, the prosthesis is an outflow graft of a ventricular assist device.

The first and second solvents may be the same or different. In a preferred embodiment, the first and second solvents are the same. The first polymer is soluble in the first solvent and the second polymer is soluble in the second solvent. The particulate is preferably insoluble in the first and second solvents. Useful solvents for use as the first or second solvent include, but are not limited to, N,N dimethyl acetamide (DMAC), N,N dimethyl formamide, tetrahydrofuran, N-methyl pyrolidone (NMP), 1,4 dioxane, dimethyl sulfoxide, and mixtures thereof. In a particularly preferred method, the first solvent is DMAC, the second solvent is DMAC and the third solvent is water.

The particulate used to form the pores in the first coat is preferably a salt, including, but not limited to, sodium chloride (NaCl), sodium bicarbonate ($NaHCO_3$), $Na_2CO_3$, $MgCl_2$, $CaCO_3$, calcium fluoride ($CaF_2$), magnesium sulfate ($MgSO_4$), $CaCl_2$, $AgNO_3$ or any water soluble salt. However, other suspended particulate materials may be used. These include, but are not limited to, sugars, polyvinyl alcohol, cellulose, gelatin or polyvinyl pyrolidone. Most preferably, the particulate is sodium chloride. Preferably, the size of the particles ranges from about 5 to about 50 microns.

The first and second polymers may be the same or different. Preferably, the first and second polymers are both a polyurethane, such as a polyurethane urea. As used herein, the term "polyurethane" includes polyurethane urea as well as other polyurethanes. Most preferably, both the first polymer and the second polymer are Thoralon® polymer.

Thoralon® polymer is a polyetherurethane urea blended with a siloxane containing surface modifying additive, and has been demonstrated to provide effective sealing of textile grafts. Thoralon® polymer may be obtained from Thoratec Corporation, Pleasanton, CA. Specifically, Thoralon® polymer is a mixture of base polymer BPS-215 and an additive SMA-300 in dimethylacetamide solvent. The concentration of additive is preferably in the range of 0.5% to 5% by weight of the base polymer.

The BPS-215 component (also available from Thoratec Corporation, Pleasanton, CA) used in Thoralon® polymer is a segmented polyether urethane urea containing a soft segment and a hard segment. The soft segment is made of polytetramethylene oxide (PTMO) and the hard segment is made of 4,4'-diphenylmethane diisocyanate (MDI) and ethylene diamine (ED).

The SMA-300 component is a polyurethane comprising polydimethylsiloxane as a soft segment and MDI and 1,4 butanediol as a hard segment. A process for synthesizing SMA-300 is described, for example, in U.S. Patent No. 4,861,830 to Ward, Jr., at Column 14, Lines 17-41, and U.S. Patent No. 4,675,361 to Ward, Jr., at Column 14, Lines 13-37.

Thoralon® polymer is FDA approved for use in certain vascular applications and has been shown to be safe and effective in a variety of critical applications because it offers thromboresistance, high tensile strength, and superb flex life. Thoralon® polymer has been shown to be biostable and useful *in vivo* in long term blood contacting applications requiring biostability and leak resistance for periods exceeding one year or more. Because of its flexibility, Thoralon® polymer is particularly beneficial in applications where elasticity and compliance are essential.

Thoralon® polymer's lower water absorption contributes to enhanced *in vivo* stability, while its lower critical surface tension and longer Lee White Clotting Times demonstrate improved blood compatibility and thromboresistance (Table 1).

**Table 1- Physical Properties of Thoralon® polymer in Comparison to Biomer**

| Physical Properties | Biomer | Thoralon® polymer |
|---|---|---|
| Water Absorption | 4.1 % wt gain | 1.8% wt. gain |
| Critical Surface Tension | 27.8 dynes/cm | 19.8 dynes/cm |
| Lee White Clotting Times | 29.1 minutes | 37 minutes |

In addition to Thoralon® polymer, other polyurethane ureas may be used as the first or second polymers in the solutions used to coat the graft. For example, BPS-215 with a capping ratio (MDI/PTMO mole ratio) ranging from about 1.0 to about 2.5 may be used. Such polyurethane ureas preferably comprise a soft segment, and a hard segment comprising a diisocyanate and diamine. For example, polyurethane ureas with soft segments such as polyethylene oxide, polypropylene oxide, polycarbonate, polyolefin, polysiloxane (e.g., polydimethylsiloxane), and other polyether soft segments made from higher homologous series of diols may be used. Mixtures of any of the soft segments may also be used. The soft segments may also have either alcohol or amine end groups. The molecular weight of the soft segment may vary from about 500 to about 5,000 g/mole, and preferably is about 2,000 g/mole.

The diisocyanate used as a component of the hard segment may be represented by the formula OCN-R-NCO. R may be aliphatic, aromatic, cycloaliphatic or aromatic-aliphatic. Representative diisocyanates useful in the invention include, but are not limited to, tetramethylene diisocyanate, hexamethylene diisocyanate, trimethyhexamethylene diisocyanate, tetramethylxylylene diisocyanate, 4,4'-decyclohexylmethane diisocyanate, dimer acid diisocyanate, isophorone diisocyanate, metaxylene diisocyanate, diethylbenzene diisocyanate, decamethylene 1,10 diisocyanate, cyclohexylene 1,2-diisocyanate, 2,4-toluene diisocyanate, 2,6-toluene diisocyanate, xylene diisocyanate, m-phenylene diisocyanate, hexahydrotolylene diisocyanate (and isomers), naphthylene-1,5-diisocyanate, 1-methoxyphenyl 2,4-diisocyanate, 4,4'-biphenylene diisocyanate, 3,3-dimethoxy-4,4'-biphenyl diisocyanate and mixtures thereof.

Suitable diamines useful as a component of the hard segment include aliphatic, aromatic and aliphatic-aromatic amines. For example, useful diamines include, but are not limited to, ethylene diamine, propane diamines, butanediamines, hexanediamines, pentane diamines, heptane diamines, octane diamines, m-xylylene diamine, cyclohexane diamines, 2-methypentamethylene diamine, 4,4'-methylene dianiline, and mixtures thereof. The amines may also contain nitrogen, oxygen or halogen.

In addition to polyurethane ureas, other polyurethanes (preferably having a chain extended with diols) may be used as the first or second polymers in the solutions used to coat the graft. Polyurethanes modified with cationic, anionic and aliphatic side chains also may be used (see, e.g., U.S. Patent No. 5,017,664 to Grasel, at Column 1, Lines 57-63, and Column 8, line 60 - Column 11, Line 27).

The soft segments of these polyurethanes may be comprised of any of the soft segments mentioned above (including, but not limited to, polytetramethylene oxide, polyethylene oxide, polypropylene oxide, polycarbonate, polyolefin, polysiloxane (e.g., polydimethylsiloxane), other polyether soft segments made from higher homologous series of diols, and mixtures and combinations of these soft segments. The soft segments may have amine or alcohol end groups).

The hard segment may be comprised of any of the diisocyantes listed above (including, but not limited to, 4,4'-diphenylmethane diisocyanate, tetramethylene diisocyanate, hexamethylene diisocyanate, trimethyhexamethylene diisocyanate, tetramethylxylylene diisocyanate, 4,4'-decyclohexylmethane diisocyanate, dimer acid diisocyanate, isophorone diisocyanate, metaxylene diisocyanate, diethylbenzene diisocyanate, decamethylene 1,10 diisocyanate, cyclohexylene 1,2-diisocyanate, 2,4-toluene diisocyanate, 2,6-toluene diisocyanate, xylene diisocyanate, m-phenylene diisocyanate, hexahydrotolylene diisocyanate (and isomers), naphthylene-1,5-diisocyanate, 1-methoxyphenyl 2,4-diisocyanate, 4,4'-biphenylene diisocyanate, 3,3-dimethoxy-4,4'-biphenyl diisocyanate and mixtures and combinations thereof).

The hard segment may be comprised of polyols. Polyols may be aliphatic, aromatic, aromatic-aliphatic or cycloaliphatic. Preferred polyols include, but are not limited to, ethylene glycol, diethylene glycol, triethylene glycol, 1,4-butanediol, neopentyl alcohol, 1,6-hexanediol, 1,8-octanediol, propylene glycols, 2,3-butylene glycol, dipropylene glycol, dibutylene glycol, glycerol, and mixtures thereof.

In addition, the polyurethanes may be end-capped with surface active end groups, such as, for example, polydimethylsiloxane, fluoropolymers, polyolefin, polyethylene oxide, or other suitable groups, including, but not limited to, those described in U.S. Patent No. 5,589,563 to Ward Jr. et al. (see, e.g., Examples 2, 3, 5 and 8, at Column 28, Line 60 - Column 31, Line 22, of U.S. Patent No. 5,589,563.

In addition to the foregoing polymers, other useful polymers for forming the first and second coats include, but are not limited to, fluoropolymers, polysulfone, acrylics, polycarbonates and cellulosics. Mixtures or combinations of any of the polymers described herein may also be used. Thoralon® polymer with and without siloxane additive (SMA) may be used.

The first and second solutions may be applied by any suitable means. Preferably, the first and second solutions are applied by placing the graft on a rod or mandrel and dipping the graft into first solution, allowing it to dry, and dipping it into the second solution. Alternately, the coatings may be individually applied by passing the graft on a mandrel through a coating die in order to apply each coating. Alternately, the coatings may be applied to the graft by dispensing the respective polymer solution onto the surface of the graft while rotating on a lathe. The first and second solutions may be applied, for example, by spraying, casting, applying with rollers or a brush.

The first coat is optimally dried by heating in an oven to between about 30°C to about 150°C. For example, the step of allowing the layer to dry may comprise heating the layer to about 50°C.

Another embodiment of the invention is directed to a method for sealing the pores of a fabric graft comprising the steps of: applying a first solution to a first surface of the graft, the first solution comprising a polyurethane and a salt in a first solvent; allowing the first solution to dry, thereby forming a first coat on the graft; applying a second solution to the coated first surface, the second solution comprising a second polyurethane in a second solvent; and immersing the coated graft in water to precipitate the second polyurethane from solution, thereby forming a porous second coat, and dissolving the salt in the first coat to form pores in the first coat. Preferably, the first and second polyurethanes are the same and the first and second solvents are the same. Most preferably, the first and second polyurethanes are Thoralon® polymer, the salt is sodium chloride and the first and second solvents are DMAC.

The invention also includes vascular prosthesis comprising the novel coatings of the invention. One such prosthesis comprises a substrate comprising a crimped woven or knitted polymer fabric, the substrate having a first surface and a second surface opposite the first surface, the second surface being a blood interface surface; a first porous coating disposed on the first surface, the first porous coating comprising a first polymer and preferably having an average pore size of 5 to 100 microns and an average porosity of between 50% to 90% by volume; and a second porous coating disposed on the first porous coating, the second porous coating comprising a second polymer and preferably having an average pore size of less than 10 microns and an average porosity of between 50% to 90% by volume. Preferably, the first coating does not penetrate to the second surface of the substrate.

The resulting prosthesis preferably has a kink resistance nearly equivalent to the uncoated substrate. Most preferably, the prosthesis has a kink diameter of about 12.7 mm (0.5 inches) or less. The prosthesis is preferably impermeable to blood and has a water permeability of less than about 0.5 ml/min/cm$^2$ at 120 mm Hg.

The fabric may be any of the fabrics discussed above, and most preferably, comprises woven or knitted polyester. The first and second polymers are preferably biocompatible and may be any of the polymers described herein, including any mixtures and combinations of them.

In a preferred embodiment, the polymers in the first and second porous coatings comprise Thoralon® polymer. However, the coatings may comprise one or more polyurethanes, or mixtures and combinations thereof. Preferably, the polyurethanes each comprise a soft segment and a hard segment. As discussed above, the soft segment may comprise one or more compounds selected from the group consisting of polytetramethylene oxide, polyethylene oxide, polypropylene oxide, polycarbonate, polyolefin, polysiloxane (e.g., polydimethylsiloxane), polyether soft segments made from higher homologous series of diols, and mixtures and combinations thereof. The soft segments may also have either alcohol or amine end groups.

The hard segment may comprise an isocyanate (preferably a diisocyanate) and an amine (preferably a diamine) or a polyol. Alternately, the hard segment may comprise an isocyanate and both an amine and a polyol. The isocyanate component of the hard segment may comprise one or more compounds selected from the group consisting of 4,4'-diphenylmethane diisocyanate (MDI), tetramethylene diisocyanate, hexamethylene diisocyanate, trimethyhexamethylene diisocyanate, tetramethylxylylene diisocyanate, 4,4'-decyclohexylmethane diisocyanate, dimer acid diisocyanate, isophorone diisocyanate, metaxylene diisocyanate, diethylbenzene diisocyanate, decamethylene 1,10 diisocyanate, cyclohexylene 1,2-diisocyanate, 2,4-toluene diisocyanate, 2,6-toluene diisocyanate, xylene diisocyanate, m-phenylene diisocyanate, hexahydrotolylene diisocyanate (and isomers), naphthylene-1,5-diisocyanate, 1-methoxyphenyl 2,4-diisocyanate, 4,4'-biphenylene diisocyanate, 3,3-dimethoxy-4,4'-biphenyl diisocyanate, and mixtures and combinations thereof. The amine component of the hard segment may comprise one or more compounds selected from the group consisting of ethylene diamine, propane diamines, butanediamines, hexanediamines, pentane diamines, heptane diamines, octane diamines, m-xylylene diamine, cyclohexane diamines, 2-methypentamethylene diamine, 4,4'-methylene dianiline, alkanol amines and diamines, and mixtures and combinations thereof. The polyol component of the hard segment may comprise one or more compounds selected from the group consisting of ethylene glycol, diethylene glycol, triethylene glycol, 1,4-butanediol, neopentyl alcohol, 1,6-hexanediol, 1,8-octanediol, propylene glycols, 2,3-butylene glycol, dipropylene glycol, dibutylene glycol, glycerol, and mixtures and combinations thereof.

Preferably, the first coating does not penetrate to the second surface of the substrate or substantially wick into the fabric (i.e., does not wick so as to negatively impact kink resistance). The second coating likewise does not penetrate to the second surface of the substrate.

In a preferred embodiment, the prosthesis is an outflow graft of a ventricular assist device. However, the invention may be broadly used, for example, as a vascular graft, a stent graft (including an AAA stent graft) or vascular patch. The prosthesis may have any desired shape, such as a cylinder, a bifurcated Y-shaped cylinder or a substantially flat sheet for patches. The invention may be used to coat large or small bore cylindrical grafts, e.g., large bore grafts having an internal diameter of 6 mm or more, or small bore grafts having an internal diameter of less than 6 mm. In one embodiment, at least a portion of prosthesis has a substantially tubular shape with an internal diameter of more than 5 mm. It may be designed to fit inside of or be anastomosed to the aorta of an adult human or another vessel such as a femoral or carotid artery.

Although preferred embodiments of the invention use two coats on the exterior of the graft (the first coat applied directly to the fabric substrate, and the second coat applied directly to the first coat), depending on the application or end use, additional coatings may be applied to the inner surface of the graft, or on top of the substrate, the first coat or the second coat without departing from the invention.

Still another embodiment of the invention is directed to a vascular prosthesis comprising: a first layer comprising a crimped woven or knitted polymer fabric having a first surface; a second layer comprising a first porous coating disposed on the first surface, the first porous coating comprising a first polymer; and a third layer comprising a second porous coating disposed on the first porous coating, the second porous coating comprising a second polymer and preferably having an average pore size of less than about 10 microns and an average porosity of between about 50% to about 90% by volume. The second layer is disposed between the first and third layers. Preferably, the first porous coating does not penetrate to a second surface of the first layer. Preferably, the prosthesis has a kink diameter of about 12.7 mm (0.5 inches) or less.

The following examples are offered to illustrate embodiments of the invention, and should not be viewed as limiting the scope of the invention.

EXAMPLES

Example 1- Materials and Methods

a. Measurement of Kink Diameter

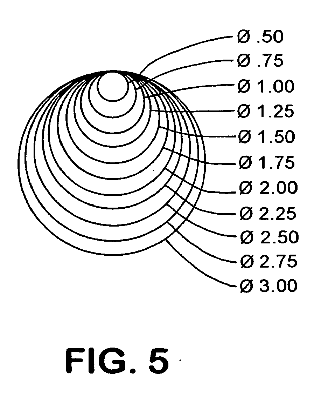

In the following Examples, the kink diameter of the different grafts was measured using a set of circular templates varying in diameter by 6.35 mm (0.25 inches). Grafts were bent around circles of decreasing diameter until a kink was created. When a kink was observed visually at the inner edge of the graft, the circle size corresponding to the inner edge of the graft was recorded. If the kink occurred between circle sizes, the circle smaller and the circle larger than the diameter at which kink occurred were both recorded. **Fig. 5** is a diagram depicting the circular template used to determine kink diameter. Measurements are in inches, not to scale.

b. Measurement of Water Permeability

Water permeability measurements were made on the grafts by sealing one end of the graft, and connecting the other end to a pressure reservoir filled with distilled water. A pressure regulator was used to adjust the pressure to 120 mm Hg. The grafts were each held over a collection container for a period of time sufficient to accurately measure the amount of water passing through the walls of the graft. The following calculation was performed for each of the 14 mm grafts tested in order to obtain the water permeability.

$$\textbf{14 mm graft} - \text{Water permeability (in ml/min/cm}^2) = \{\text{ml water} \div \text{time (min.)}\} \div$$
$$\{\text{graft length (cm) x 4.40}\}$$

Example 2- Properties of the Base Graft

The base graft used in Examples 3-8 was a woven polyester graft available from Boston Scientific/Medi-Tech (Natick, MA), having an internal diameter of 14 mm. The measured kink diameter for the uncoated base graft was 6.35 to 12.7mm (0.25 to 0.5 inches). The measured water permeability was 30 to 45 ml/min/cm$^2$ at 120 mm Hg.

Example 3- Graft with Evaporated Thoralon® polymer Coating

A 14 mm graft was placed on a stainless steel rod and dipped into a solution of 8% by weight Thoralon® polymer in DMAC. The graft was withdrawn from the solution at a fixed rate, resulting in deposition of solution on the outside of the graft. The coated graft was suspended in a forced air oven at 50°C for 3 hours in order to evaporate the solvent.
The kink diameter occurred between 19.05mm and 25.4mm (0.75 and 1.0 inches). There was no measurable water permeability through the graft after 5 minutes at 120 mm Hg. There was obvious permeation of the Thoralon® polymer into the wall of the fabric. This contributed to the increase in kink diameter over the starting graft material.

Example 4- Graft with Precipitated Thoralon® polymer Coating

A 14 mm graft was placed on a stainless steel rod and dipped into a solution of 8% by weight Thoralon® polymer in DMAC. The graft was withdrawn from the solution at a fixed rate, resulting in deposition of solution on the outside of the graft. The coated graft was immediately immersed into water, thereby precipitating the Thoralon® polymer. The coated graft was water washed for 1 hour and dried in a 50° oven for 1 hour.
The kink diameter occurred between 12.7mm and 19.05mm (0.5 and 0.75 inches). The water permeability was measured to be 1.0 ml/min/cm$^2$ at 120 mm Hg. As in the previous example, the kink diameter was increased significantly over the starting graft material.

Example 5- Graft Coated with Salt Containing Thoralon® polymer Solution

A 14 mm graft was placed on a stainless steel rod and dipped into a solution containing 7.3% by weight Thoralon® polymer, 50.6% by weight dimethylacetamide (DMAC), and 42.1% by weight sodium chloride. The graft was withdrawn from the solution at a fixed rate, resulting in deposition of solution on the outside of the graft. The coated graft was suspended in a forced air oven at 50°C for 45 minutes in order to evaporate the solvent. The graft was removed from the stainless steel rod and placed into a 60°C water bath for 16 hours in order to dissolve the salt particles and remove residual DMAC. The graft was then dried in a 50°C oven for 1 hour.

The kink diameter occurred at 12.7mm (0.5 inches). The water permeability was measured to be 8.4 ml/min/cm$^2$ at 120 mm Hg. This graft was tested *in vitro* with pig blood, and it was found that the blood freely permeated through the wall of the graft.

Example 6 - Graft Coated with Salt Containing Thoralon® polymer Solution Followed by Coating and Precipitating Thoralon Solution

A 14 mm graft was placed on a stainless steel rod and dipped into a solution containing 7.3% Thoralon® polymer, 50.6% dimethylacetamide (DMAC), and 42.1% sodium chloride. The graft was withdrawn from the solution at a fixed rate, resulting in deposition of solution on the outside of the graft. The coated graft was suspended in a forced air oven at 50°C for 45 minutes in order to evaporate the solvent. The coated graft was then dipped into an 8% Thoralon® polymer in DMAC solution, and again withdrawn at a fixed rate. Immediately after withdrawing the graft from the Thoralon® polymer solution, the graft was rapidly immersed into water in order to precipitate the Thoralon® polymer. After 15 minutes, the graft was removed from the stainless steel rod and placed into a 60°C water bath for 16 hours in order to dissolve the salt particles and remove residual DMAC. The graft was then dried in a 50° C oven for 1 hour.

The kink diameter occurred at 12.7mm (0.5 inches). The water permeability was measured to be 0.33 ml/min/cm$^2$ at 120 mm Hg.

The graft of this example was implanted as an aortic bypass in four anticoagulated calves. Visual observation of the graft following implantation and post-operative follow up confirmed the graft was blood impermeable.

Table 2 shows a comparison of the grafts of Examples 2, 4, 5 and 6. As is clear from the data, the graft coated with both the salt containing Thoralon® polymer solution followed by the precipitated Thoralon® polymer demonstrated low permeability and good resistance to kinking.

**Table 2- Comparative Results**

| Graft Material | Water Permeability (at 120 mm Hg) | Blood Permeability | Kink Diameter |
|---|---|---|---|
| uncoated graft | 30-45 ml/min.cm$^2$ | -------- | (0.25-.5 inches) 12.7mm |
| graft coated with precipitated Thoralon® polymer solution | 1.0 ml/min.cm$^2$ | --------- | (0.5 - 0.75 inches) 12.7 - 19.05 mm |
| graft coated with salt containing solution | 8.4 ml/min.cm$^2$ | permeable | (0.5 inches) |
| graft coated with salt containing solution followed by coating with Thoralon® polymer solution and precipitating | 0.33 ml/min.cm$^2$ | impermeable | (0.5 inches) |

Example 7 - Graft Coated with Lower Salt Content Thoralon® polymer Solution

The same procedure was used in this example as with the previous example, except the first dipping solution contained 6.5% Thoralon® polymer, 55.9% DMAC, and 37.6% sodium chloride.

The kink diameter occurred between 19.05mm and 25.4mm (0.75 and 1.0 inches). The water permeability was measured to be 0.24 ml/min/cm$^2$ at 120 mm Hg. In this case, the kink diameter was increased over the starting material, due to the permeation of the salt containing solution into the fabric.

Example 8 - Graft Coated with Higher Salt Content Thoralon® polymer Solution

The same procedure was used in this example as in the previous example, except the first dipping solution contained 4.1 % Thoralon® polymer, 40.8% DMAC, and 55.1% sodium chloride.

The kink diameter occurred at 12.7mm (0.5 inches). In attempting to measure the water permeability, the Thoralon® polymer coating on the outside of the graft delaminated under the applied pressure of the test. In this case, the higher sodium chloride content prevented the solution from permeating into the fabric, such that there was no adhesion of the coating to the fabric.

Although the invention has been described with respect to preferred embodiments, the foregoing description and examples are intended to be merely exemplary of the invention. It will be apparent that certain agents which are both chemically and physiologically related may be substituted for the agents described herein while the same or similar results would be achieved. In addition to vascular applications, the invention may be modified for use in other types of grafts.

## Claims

1. A method for making a vascular prosthesis comprising the steps of:

   providing a crimped substrate, the substrate comprising a woven or knitted polymer fabric, the substrate having a first surface and a second surface opposite the first surface, the second surface being a blood interface surface;
   coating the first surface with a first solution, the first solution comprising a first polymer and a particulate in a first solvent;
   allowing the first solution to dry, thereby forming a first coat on the substrate;
   coating the first coat with a second solution, the second solution comprising a second polymer in a second solvent; and
   immersing the coated substrate in a third solvent to precipitate the second polymer from solution, thereby forming a porous second coat;
   wherein the particulate is soluble in the third solvent and the step of immersing in the third solvent dissolves the particulate in the first coat, thereby causing pores to form in the first coat.

2. The method of claim 1 wherein the particulate in the first solution is present in an amount which inhibits wicking of the first solution into the fabric.

3. The method of claim 1 wherein the prosthesis has a kink diameter of about 12.7 mm (0.5 inches) or less.

4. The method of claim 1 wherein the prosthesis is impermeable to blood.

5. The method of claim 2 wherein the first solution only penetrates the first surface of the graft.

6. The method of claim 1 wherein the prosthesis has a water permeability of less than about 0.5 ml/min/cm$^2$ at 120 mm Hg.

7. The method of claim 1 wherein the fabric is selected from the group consisting of Dacron® material, Teflon® material, PTFE, and polyester.

8. The method of claim 1 wherein the prosthesis comprises at least one device selected from the group consisting of an outflow graft of a ventricular assist device, a vascular graft, a stent graft, and a vascular patch.

9. The method of claim 1 wherein the first and the second solvents are the same.

10. The method of claim 9 wherein the first solvent is DMAC, the second solvent is DMAC, and the third solvent is water.

11. The method of claim 1 wherein the particulate is a salt.

12. The method of claim 11 wherein the salt is selected from the group consisting of NaCl, NaHCO$_3$, Na$_2$CO$_3$, MgCl$_2$, CaCO$_3$ CaF$_2$, MgSO$_4$, CaCl$_2$, and AgNO$_3$.

13. The method of claim 1 wherein the first and the second polymers are the same.

14. The method of claim 1 wherein the first polymer is at least one polymer selected from the group consisting of a polyurethane, a polyurethane urea, and a Thoralon® polymer, and wherein the second polymer is at least one polymer selected from the group consisting of a polyurethane, a polyurethane urea, and a Thoralon® polymer.

15. The method of claim 14 wherein the each of the first polymer and the second polymer comprises a soft segment and a hard segment.

16. The method of claim 15 wherein the soft segment comprises one or more compounds selected from the group consisting of polyethylene oxide, polypropylene oxide, polytetramethylene oxide, polycarbonate, polyolefin, polysiloxane, polyether soft segments made from higher homologous series of diols, and mixtures and combinations thereof, and the hard segment comprises one or more compounds selected from the group consisting of 4,4'-diphenylmethane diisocyanate, tetramethylene diisocyanate, hexamethylene diisocyanate, trimethyhexamethylene diisocyanate, tetramethylxylylene diisocyanate, 4,4'-decyclohexylmethane diisocyanate, dimer acid diisocyanate, isophorone diisocyanate, metaxylene diisocyanate, diethylbenzene diisocyanate, decamethylene 1,10 diisocyanate, cyclohexylene 1,2-diisocyanate, 2,4-toluene diisocyanate, 2,6-toluene diisocyanate, xylene diisocyanate, m-phenylene diisocyanate, hexahydrotolylene diisocyanate (and isomers), naphthylene-1,5-diisocyanate, 1-methoxyphenyl 2,4-diisocyanate, 4,4'-biphenylene diisocyanate, 3,3-dimethoxy-4,4'-biphenyl diisocyanate, ethylene diamine, propane diamines, butanediamines, hexanediamines, pentane diamines, heptane diamines, octane diamines, m-xylylene diamine, cyclohexane diamines, 2-methypentamethylene diamine, 4,4'-methylene dianiline, alkanol amines and diamines, ethylene glycol, diethylene glycol, triethylene glycol, 1,4-butanediol, neopentyl alcohol, 1,6-hexanediol, 1,8-octanediol, propylene glycols, 2,3-butylene glycol, dipropylene glycol, dibutylene glycol, glycerol, and mixtures and combinations thereof.

17. The method of claim 1 wherein the step of coating with the first solution comprises placing the substrate on a rod and dipping the substrate into the first solution, and wherein the step of coating with the second solution comprises dipping the substrate into the second solution after the first solution has been allowed to dry.

18. The method of claim 1 wherein the step of allowing the first solution to dry comprises heating to between 30°C to 150°C.

19. A method for sealing the pores of a fabric graft comprising the steps of:

applying a first solution to a first surface of the graft, the first solution comprising a polyurethane and a salt in a first solvent;
allowing the first solution to dry, thereby forming a first coat on the graft;
applying a second solution to the coated first surface, the second solution comprising a second polyurethane in a second solvent; and
immersing the coated graft in water to precipitate the second polyurethane from solution, thereby forming a porous second coat, and dissolving the salt in the first coat to form pores in the first coat.

20. The method of claim 19 wherein the salt is present in an amount which inhibits wicking of the first solution into the fabric graft.

21. The method of claim 20 wherein the first solution only penetrates the first surface of the graft.

22. The method of claim 19 wherein the first and the second polyurethanes are the same and the first and the second solvents are the same.

23. The method of claim 19 wherein the first and the second polyurethanes are Thoralon® polymer, the salt is sodium chloride, and the first and the second solvents are DMAC.

24. A vascular prosthesis comprising:

a substrate comprising a crimped woven or knitted polymer fabric, the substrate having a first surface and a second surface opposite the first surface, the second surface being a blood interface surface;
a first porous coating disposed on the first surface, the first porous coating comprising a first polymer; and
a second porous coating disposed on the first porous coating, the second porous coating comprising a second polymer, and wherein the first coating does not penetrate to the second surface of the substrate.

25. The prosthesis of claim 24 wherein the prosthesis is impermeable to blood.

**26.** The prosthesis of claim 24 wherein the prosthesis has a water permeability of less than about 0.5 ml/min/cm$^2$ at 120 mm Hg.

**27.** The prosthesis of claim 24 wherein the fabric is selected from the group consisting of Dacron® material, Teflon® material, PTFE, and polyester.

**28.** The prosthesis of claim 24 wherein the second coating only penetrates the first surface of the substrate.

**29.** The prosthesis of claim 24 wherein the prosthesis comprises at least one device selected from the group consisting of an outflow graft of a ventricular assist device, a vascular graft, a stent graft, and a vascular patch.

**30.** The prosthesis of claim 24 wherein at least a portion of prosthesis has a substantially tubular shape.

**31.** The prosthesis of claim 30 wherein the substrate has an internal diameter of more than 5mm.

**32.** The prosthesis of claim 24 wherein the first polymer is at least one polymer selected from the group consisting of a polyurethane, a polyurethane urea, and a Thoralon® polymer, and wherein the second polymer is at least one polymer selected from the group consisting of a polyurethane, a polyurethane urea, and a Thoralon® polymer.

**33.** The prosthesis of claim 32 wherein each of the first polymer and the second polymer comprises a soft segment and a hard segment.

**34.** The prosthesis of claim 33 wherein the soft segment has a molecular weight of about 2,000 g/mole.

**35.** The prosthesis of claim 33 wherein the soft segment comprises one or more compounds selected from the group consisting of polyethylene oxide, polypropylene oxide, polytetramethylene oxide, polycarbonate, polyolefin, polysiloxane, polyether soft segments made from higher homologous series of diols, and mixtures and combinations thereof.

**36.** The prosthesis of claim 33 wherein the hard segment comprises one or more compounds selected from the group consisting of 4,4'-diphenylmethane diisocyanate, tetramethylene diisocyanate, hexamethylene diisocyanate, trimethyhexamethylene diisocyanate, tetramethylxylylene diisocyanate, 4,4'-decyclohexylmethane diisocyanate, dimer acid diisocyanate, isophorone diisocyanate, metaxylene diisocyanate, diethylbenzene diisocyanate, decamethylene 1,10 diisocyanate, cyclohexylene 1,2-diisocyanate, 2,4-toluene diisocyanate, 2,6-toluene diisocyanate, xylene diisocyanate, m-phenylene diisocyanate, hexahydrotolylene diisocyanate (and isomers), naphthylene-1,5-diisocyanate, 1-methoxyphenyl 2,4-diisocyanate, 4,4'-biphenylene diisocyanate, 3,3-dimethoxy-4,4'-biphenyl diisocyanate, ethylene diamine, propane diamines, butanediamines, hexanediamines, pentane diamines, heptane diamines, octane diamines, m-xylylene diamine, cyclohexane diamines, 2-methypentamethylene diamine, 4,4'-methylene dianiline, alkanol amines and diamines, ethylene glycol, diethylene glycol, triethylene glycol, 1,4-butanediol, neopentyl alcohol, 1,6-hexanediol, 1,8-octanediol, propylene glycols, 2,3-butylene glycol, dipropylene glycol, dibutylene glycol, glycerol, and mixtures and combinations thereof.

**37.** A vascular prosthesis comprising:

a first layer comprising a crimped woven or knitted polymer fabric having a first surface;
a second layer comprising a first porous coating disposed on the first surface, the first porous coating comprising a first polymer; and
a third layer comprising a second porous coating disposed on the first porous coating, the second porous coating comprising a second polymer, and wherein the second layer is disposed between the first and third layers, and wherein the first porous coating does not penetrate to a second surface of the first layer.

**38.** The prosthesis of claim 24 or 37 wherein the second porous coating has an average pore size of less than 10 microns and an average porosity of between 50% to 90% by volume.

**39.** The prosthesis of claim 24 or 37 wherein the prosthesis has a kink diameter of about 12.7 mm (0.5 inches) or less.

**Patentansprüche**

1. Ein Verfahren zur Herstellung einer vaskulären Prothese, umfassend die Schritte:

   Bereitstellen eines gefalteten Substrats, wobei das Substrat ein gewebtes oder gestricktes Polymergewebe umfasst, wobei das Substrat eine erste Oberfläche und eine zweite Oberfläche gegenüber der ersten Oberfläche hat, wobei die zweite Oberfläche eine Blutschnittstellenoberfläche ist;
   Beschichten der ersten Oberfläche mit einer ersten Lösung, wobei die erste Lösung ein erstes Polymer und ein Teilchen in einem ersten Lösungsmittel umfasst;
   Trocknen lassen der ersten Lösung, wobei eine erste Schicht auf dem Substrat gebildet wird;
   Beschichten der ersten Schicht mit einer zweiten Lösung, wobei die zweite Lösung ein zweites Polymer in einem zweiten Lösungsmittel umfasst; und
   Eintauchen des beschichteten Substrats in ein drittes Lösungsmittel, um das zweite Polymer aus der Lösung zu fällen, wodurch eine poröse zweite Schicht gebildet wird;
   wobei das Teilchen in dem dritten Lösungsmittel löslich ist und der Schritt des Eintauchens in das dritte Lösungsmittel das Teilchen in der ersten Schicht auflöst, wodurch die Bildung von Poren in der ersten Schicht verursacht wird.

2. Das Verfahren nach Anspruch 1, wobei das Teilchen in der ersten Lösung in einer Menge vorliegt, die den Dochteffekt der ersten Lösung in dem Gewebe hemmt.

3. Das Verfahren nach Anspruch 1, wobei die Prothese einen Knickdurchmesser von etwa 12,7 mm (0,5 Zoll) oder weniger hat.

4. Das Verfahren nach Anspruch 1, wobei die Prothese impermeabel für Blut ist.

5. Das Verfahren nach Anspruch 2, wobei die erste Lösung nur in die erste Oberfläche des Transplantats eindringt.

6. Das Verfahren nach Anspruch 1, wobei die Prothese eine Wasserpermeabilität von weniger als etwa 0,5 ml/Min/cm$^2$ bei 120 mm Hg hat.

7. Das Verfahren nach Anspruch 1, wobei das Gewebe ausgewählt ist aus Dacron® Material, Teflon® Material, PTFE und Polyester.

8. Das Verfahren nach Anspruch 1, wobei die Prothese mindestens eine Vorrichtung umfasst, die ausgewählt ist aus einem Abflusstransplantat einer ventrikulären Hilfsvorrichtung, einem vaskulären Transplantat, einem Stent-Transplantat und einem vaskulären Pflaster.

9. Das Verfahren nach Anspruch 1, wobei das erste und das zweite Lösungsmittel das gleiche ist.

10. Das Verfahren nach Anspruch 9, wobei das erste Lösungsmittel DMAC ist, das zweite Lösungsmittel DMAC ist und das dritte Lösungsmittel Wasser ist.

11. Das Verfahren nach Anspruch 1, wobei das Teilchen ein Salz ist.

12. Das Verfahren nach Anspruch 11, wobei das Salz ausgewählt ist aus NaCl, NaHCO$_3$, Na$_2$CO$_3$, MgCl$_2$, CaCO$_3$, CaF$_2$, MgSO$_4$, CaCl$_2$ und AgNO$_3$.

13. Das Verfahren nach Anspruch 1, wobei das erste und das zweite Polymer das gleiche ist.

14. Das Verfahren nach Anspruch 1, wobei das erste Polymer mindestens ein Polymer ist, das ausgewählt ist aus einem Polyurethan, einem Polyurethan-Harnstoff und einem Thoralon®-Polymer, und wobei das zweite Polymer mindestens ein Polymer ist, das ausgewählt ist aus einem Polyurethan, einem Polyurethan-Harnstoff und einem Thoralon®-Polymer.

15. Das Verfahren nach Anspruch 14, wobei jedes des ersten Polymers und des zweiten Polymers ein weiches Segment und ein hartes Segment umfasst.

**16.** Das Verfahren nach Anspruch 15, wobei das weiche Segment eine oder mehrere Verbindungen umfasst, die ausgewählt sind aus Polyethylenoxid, Polypropylenoxid, Polytetramethylenoxid, Polycarbonat, Polyolefin, Polysiloxan, weiche Polyether-Segmente, die aus höher homologen Serien von Diolen hergestellt sind und Gemische und Kombinationen davon, und das harte Segment eine oder mehrere Verbindungen umfasst, die ausgewählt sind aus 4,4'-Diphenylmethandiisocyanat, Tetramethylendiisocyanat, Hexamethylendiisocynat, Trimethylhexamethylendiisocyanat, Tetramethylxylylendiisocyanat, 4,4'-Decyclohexylmethandiissocyanat, dimerem Säurediisocyanat, Isophorondiisocyanat, Metaxylendiisocyanat, Diethylbenzendiisocyanat, Decamethylen-1,10-Diisocyanat, Cyclohexylen-1,2-Diisocyanat, 2,4-Toluendiisocyanat, 2,6-Toluendiisocyanat, Xylendiisocyanat, m-Phenylendiisocyanat, Hexahydrotolylendiisocyanat (und Isomere), Naphthylen-1,5-diisocyanat, 1-Methoxyphenyl 2,4-diisocyanat, 4,4-Biphenylendiisocyanat, 3,3-Dimethoxy-4,4'-Biphenyldiisocyanat, Ethylendiamin, Propandiamine, Butandiamine, Hexandiamine, Pentandiamine, Heptandiamine, Oktandiamine, m-Xylylendiamin, Cyclohexandiamine, 2-Methylpentamethylendiamin, 4,4'-Mehylendianilin, Alkanolamine und -diamine, Ethylenglycol, Diethylenglycol, Triethylenglycol, 1,4-Butandiol, Neopentylallcohol, 1,6-Hexandiol, 1,8-Octandiol, Propylenglycol, 2,3-Butylenglycol, Dipropylenglycol, Dibutylenglycol, Glycerin und Gemische und Kombinationen davon.

**17.** Das Verfahren nach Anspruch 1, wobei der Schritt des Beschichtens mit der ersten Lösung das Aufbringen des Substrats auf einen Stab und das Eintauchen des Substrats in die erst Lösung umfasst, und wobei der Schritt des Beschichtens mit der zweiten Lösung das Eintauchen des Substrats in die zweite Lösung umfasst, nachdem der ersten Lösung erlaubt wurde zu trocknen.

**18.** Das Verfahren nach Anspruch 1, wobei der Schritt des Trocknens der ersten Lösung ein Erhitzen auf zwischen 30°C bis 150°C umfasst.

**19.** Ein Verfahren zum Abdichten der Poren eines Gewebetransplantats, umfassend die Schritte:

Anwenden einer ersten Lösung auf einer ersten Oberfläche des Transplantats, wobei die erste Lösung ein Polyurethan und ein Salz in einem ersten Lösungsmittel umfasst;
Trocknen der ersten Lösung, wobei eine erste Schicht auf dem Transplantat gebildet wird;
Anwenden einer zweiten Lösung auf der beschichteten ersten Oberfläche, wobei die zweite Lösung ein zweites Polyurethan in einem zweiten Lösungsmittel umfasst; und
Eintauchen des beschichteten Transplantats in Wasser, um das zweite Polyurethan aus der Lösung zu fällen, wobei eine poröse zweite Schicht gebildet wird, und Auflösen des Salzes in der ersten Schicht, um Poren in der ersten Schicht zu bilden.

**20.** Das Verfahren nach Anspruch 19, wobei das Salz in einer Menge vorliegt, die den Dochteffekt der ersten Lösung in dem Gewebetransplantat hemmt.

**21.** Das Verfahren nach Anspruch 20, wobei die erste Lösung nur in die erste Oberfläche des Transplantats eindringt.

**22.** Das Verfahren nach Anspruch 19, wobei das erste und das zweite Polyurethan das gleiche ist und das erste und das zweite Lösungsmittel das gleiche ist.

**23.** Das Verfahren nach Anspruch 19, wobei das erste und das zweite Polyurethan Thoralon®-Polymer ist, das Salz Natriumchlorid ist und das erste und das zweite Lösungsmittel DMAC ist.

**24.** Eine vaskuläre Prothese umfassend:

Ein Substrat umfassend ein gefaltetes, gewebtes oder gefaltetes, gestricktes Polymergewebe, wobei das Substrat eine erste Oberfläche und eine zweite Oberfläche gegenüber der ersten Oberfläche hat, wobei die zweite Oberfläche eine Blutschnittstellenoberfläche ist;
eine erste poröse Schicht, die auf der ersten Oberfläche angeordnet ist, wobei die erste poröse Schicht ein erstes Polymer umfasst; und
eine zweite poröse Schicht, die auf der ersten porösen Schicht angeordnet ist, wobei die zweite poröse Schicht ein zweites Polymer umfasst, und wobei die erste Schicht nicht in die zweite Oberfläche des Substrats eindringt.

**25.** Die Prothese nach Anspruch 24, wobei die Prothese impermeabel für Blut ist.

**26.** Die Prothese nach Anspruch 24, wobei die Prothese eine Wasserpermeabilität von weniger als etwa 0,5 ml/Min/cm$^2$

bei 120mm Hg hat.

**27.** Die Prothese nach Anspruch 24, wobei das Gewebe ausgewählt ist aus Dacron®-Material, Teflon®-Material, PTFE und Polyester.

**28.** Die Prothese nach Anspruch 24, wobei die zweite Schicht nur in die erste Oberfläche des Substrats eindringt.

**29.** Die Prothese nach Anspruch 24, wobei die Prothese mindestens eine Vorrichtung umfasst, die ausgewählt ist aus einem Abflusstransplantat einer ventrikulären Hilfsvorrichtung, einem vaskulären Transplantat, einem Stent-Transplantat und einem vaskulären Pflaster.

**30.** Die Prothese nach Anspruch 24, wobei mindestens ein Teil der Prothese eine im Wesentlichen röhrenförmige Form hat.

**31.** Die Prothese nach Anspruch 30, wobei das Substrat einen Innendurchmesser von mehr als 5 mm hat.

**32.** Die Prothese nach Anspruch 24, wobei das erste Polymer mindestens ein Polymer ist, das ausgewählt ist aus einem Polyurethan, einem Polyurethan-Harnstoff und einem Thoralon®-Polymer, und wobei das zweite Polymer mindestens ein Polymer ist, das ausgewählt ist aus einem Polyurethan, einem Polyurethan-Harnstoff und einem Thoralon®-Polymer.

**33.** Die Prothese nach Anspruch 32, wobei jedes des erstes Polymers und des zweiten Polymers ein weiches Segment und ein hartes Segment umfasst.

**34.** Die Prothese nach Anspruch 33, wobei das weiche Segment ein Molelculargewicht von etwa 2.000 g/mol hat.

**35.** Die Prothese nach Anspruch 33, wobei das weiche Segment eine oder mehrere Verbindungen umfasst, die ausgewählt sind aus Polyethylenoxid, Polypropylenoxid, Polytetramethylenoxid, Polycarbonat, Polyolefin, Polysiloxan, weiche Polyether-Segmente, die aus höher homologen Serien von Diolen hergestellt sind und Gemische und Kombinationen davon.

**36.** Die Prothese nach Anspruch 33, wobei das harte Segment eine oder mehrere Verbindungen umfasst, die ausgewählt sind aus 4,4'-Diphenylmethandiisocyanat, Tetramethylendiisocyanat, Hexamethylendiisocynat, Trimethylhexamethylendiisocyanat, Tetramethylxylylendiisocyanat, 4,4'-Dccyclohexylmethandiissocyanat, dimerem Säurediisocyanat, Isophorondiisocyanat, Metaxylendiisocyanat, Diethylbenzendiisocyanat, Decamethylen-1,10-Diisocyanat, Cyclohexylen-1,2-Diisocyanat, 2,4-Toluendiisocyanat, 2,6-Toluendiisocyanat, Xylendiisocyanat, m-Phenylendiisocyanat, Hexahydrotolylendiisocyanat (und Isomere), Naphthylen-l,5-diisocyanat, 1-Methoxyphenyl 2,4-diisocyanat, 4,4-Biphenylendiisocyanat, 3,3-Dimethoxy-4,4'-Biphenyldiisocyanat, Ethylendiamin, Propandiamine, Butandiamine, Hexandiamine, Pentandiamine, Heptandiamine, Oktandiamine, m-Xylylendiamin, Cyclohexandiamine, 2-Methylpentamethylendiamin, 4,4'-Mehylendianilin, Alkanolamine und -diamine, Ethylenglycol, Diethylenglycol, Triethylenglycol, 1,4-Butandiol, Neopentylallcohol, 1,6-Hexandiol, 1,8-Octandiol, Propylenglycol, 2,3-Butylenglycol, Dipropylenglycol, Dibutylenglycol, Glycerin und Gemische und Kombinationen davon.

**37.** Eine vaskuläre Prothese umfassend:

Eine erste Lage, umfassend ein gefaltetes, gewebtes oder gefaltetes, gestricktes Polymergewebe, das eine erste Oberfläche hat;
eine zweite Lage, umfassend eine erste poröse Schicht, die auf der ersten Oberfläche angeordnet ist, wobei die erste poröse Schicht ein erstes Polymer umfasst; und
eine dritte Lage, umfassend eine zweite poröse Schicht, die auf der ersten porösen Schicht angeordnet ist, wobei die zweite poröse Schicht ein zweites Polymer umfasst, und wobei die zweite Lage zwischen der ersten und dritten Lage angeordnet ist, und wobei die erste poröse Schicht nicht bis zu einer zweiten Oberfläche der ersten Lage eindringt.

**38.** Die Prothese nach Anspruch 24 oder 37, wobei die zweite poröse Schicht eine Durchschnittsporengröße von weniger als 10 Mikrometer und eine Durchschnittsporosität von zwischen 50% bis 90% des Volumens hat.

**39.** Die Prothese nach Anspruch 24 oder 37, wobei die Prothese einen Knickdurchmesser von etwa 12,7 mm (0,5 Zoll) oder weniger hat.

## Revendications

1.  Procédé pour fabriquer une prothèse vasculaire comprenant les étapes consistant à :

    prévoir un substrat ondulé, le substrat comprenant un tissu polymère tissé ou tricoté, le substrat ayant une première surface et une seconde surface opposée à la première surface, la seconde surface étant une surface d'interface de sang ;
    recouvrir la première surface avec une première solution, la première solution comprenant un premier polymère et une particule dans un premier solvant ;
    permettre à la première solution de sécher, formant ainsi un premier revêtement sur le substrat ;
    recouvrir le premier revêtement avec une seconde solution, la seconde solution comprenant un second polymère dans un deuxième solvant ; et
    immerger le substrat recouvert dans un troisième solvant pour précipiter le second polymère de la solution, formant ainsi un second revêtement poreux ;
    dans lequel la particule est soluble dans le troisième solvant et l'étape d'immersion dans le troisième solvant dissout la particule dans le premier revêtement, amenant ainsi des pores à se former dans le premier revêtement.

2.  Procédé selon la revendication 1, dans lequel la particule dans la première solution est présente selon une quantité qui empêche l'effet de mèche de la première solution dans le tissu.

3.  Procédé selon la revendication 1, dans lequel la prothèse a un diamètre de vrille d'environ 12,7 mm (0,5 pouces) ou moins.

4.  Procédé selon la revendication 1, dans lequel la prothèse est imperméable au sang.

5.  Procédé selon la revendication 2, dans lequel la première solution ne pénètre que dans la première surface de la greffe.

6.  Procédé selon la revendication 1, dans lequel la prothèse a une perméabilité à l'eau inférieure à environ 0,5 ml/mn/cm$^2$ à 120 mm Hg.

7.  Procédé selon la revendication 1, dans lequel le tissu est choisi dans le groupe comprenant le Dacron®, le Teflon®, le PTFE et le polyester.

8.  Procédé selon la revendication 1, dans lequel la prothèse comprend au moins un dispositif choisi dans le groupe comprenant une greffe de sortie d'un dispositif d'assistance ventriculaire, une greffe vasculaire, une greffe de stent et un patch vasculaire.

9.  Procédé selon la revendication 1, dans lequel les premier et deuxième solvants sont identiques.

10. Procédé selon la revendication 9, dans lequel le premier solvant est du DMAC, le deuxième solvant est du DMAC et le troisième solvant est de l'eau.

11. Procédé selon la revendication 1, dans lequel la particule est un sel.

12. Procédé selon la revendication 11, dans lequel le sel est choisi dans le groupe contenant NaCl, NaHCO$_3$, Na$_2$CO$_3$, MgCl$_2$, CaCO$_3$, CaF$_2$, MgSO$_4$, CaCl$_2$ et AgNO$_3$.

13. Procédé selon la revendication 1, dans lequel les premier et second polymères sont les mêmes.

14. Procédé selon la revendication 1, dans lequel le premier polymère est au moins un polymère choisi dans le groupe comprenant un polyuréthane, une urée de polyuréthane et un polymère Thoralon®, et dans lequel le second polymère est au moins un polymère choisi dans le groupe comprenant un polyuréthane, une urée de polyuréthane et un polymère Thoralon®.

**15.** Procédé selon la revendication 14, dans lequel chacun parmi le premier polymère et le second polymère comprend un segment souple et un segment dur.

**16.** Procédé selon la revendication 15, dans lequel le segment souple comprend un ou plusieurs composés choisis dans le groupe comprenant l'oxyde de polyéthylène, l'oxyde de polypropylène, l'oxyde de polytétraméthylène, le polycarbonate, le polyoléfine, le polysiloxane, des segments souples de polyéther fabriqués à partir d'une série homologue supérieure de diols, et de ses mélanges et de ses combinaisons, et le segment dur comprend un ou plusieurs composés choisis dans le groupe comprenant le diisocyanate 4,4'-diphénylméthane, le diisocyanate de tétraméthylène, le diisocyanate d'hexaméthylène, le diisocyanate de triméthyl hexaméthylène, le diisocyanate 4,4'-dicyclohexyl méthane, le diisocyanate d'acide dimérisé, le diisocyanate d'isophorone, le diisocyanate de méthaxylène, le diisocyanate de diéthylbenzène, le 1,10-diisocyanate de décaméthylène, le 1,2-diisocyanate de cyclohexylène, le diisocyanate de 2,4-toluène, le diisocyanate de 2,6-toluène, le diisocyanate de xylène, le diisocyanate de m-phénylène, le diisocyanate, d'hexahydrotolylène (et ses isomères), le 1,5-diisocyanate de naphtylène, le 2,4-diisocyanate de 1-méthoxyphényle, le diisocyanate de 4,4'-biphénylène, le diisocyanate de 3,3-diméthoxy-4,4'-biphényle, l'éthylène diamine, les propane diamines, les butane diamines, les hexane diamines, les pentane diamines, les heptane diamines, les octane diamines, la m-xylylène diamine, les cyclohexane diamines, la 2-méthylpentaméthylène diamine, la 4,4'-méthylène dianiline, les alcanol amines et diamines, l'éthylène glycol, le diéthylène glycol, le triéthylène glycol, le 1,4-butanediol, l'alcool néopentylique, le 1,6-hexanediol, le 1,8-octanediol, les propylène glycols, le 2,3-butylène glycol, le dipropylène glycol, le dibutylène glycol, le glycérol, et des mélanges et des combinaisons de ceux-ci.

**17.** Procédé selon la revendication 1, dans lequel l'étape consistant à recouvrir avec une première solution comprend l'étape consistant à placer le substrat sur une tige et à plonger le substrat dans la première solution, et dans lequel l'étape consistant à recouvrir avec la seconde solution comprend l'étape consistant à plonger le substrat dans la seconde solution après que la première solution a été autorisée à sécher.

**18.** Procédé selon la revendication 1, dans lequel l'étape consistant à permettre à la première solution de sécher comprend l'étape consistant à la chauffer à une température comprise entre 30 °C et 150 °C.

**19.** Procédé pour réaliser l'étanchéité des pores d'une greffe en tissu comprenant les étapes consistant à :

appliquer une première solution sur une première surface de la greffe, la première solution comprenant un polyuréthane et un sel dans un premier solvant ;
permettre à la première solution de sécher, formant ainsi un premier revêtement sur la greffe ;
appliquer une seconde solution sur la première surface recouverte, la seconde solution comprenant un second polyuréthane dans un deuxième solvant ; et
immerger la greffe recouverte dans l'eau afin de précipiter le second polyuréthane de la solution, formant ainsi un second revêtement poreux, et dissolvant le sel dans le premier revêtement afin de former des pores dans le premier revêtement.

**20.** Procédé selon la revendication 19, dans lequel le sel est présent selon une quantité qui empêche l'effet de mèche de la première solution dans la greffe en tissu.

**21.** Procédé selon la revendication 20, dans lequel la première solution ne pénètre que dans la première surface de la greffe.

**22.** Procédé selon la revendication 19, dans lequel le premier et le second polyuréthane sont identiques et le premier et le deuxième solvant sont identiques.

**23.** Procédé selon la revendication 19, dans lequel le premier et le second polyuréthane sont un polymère de Thoralon®, le sel est du chlorure de sodium et le premier et le deuxième solvant sont du DMAC.

**24.** Prothèse vasculaire comprenant :

un substrat comprenant un tissu polymère ondulé tissé ou tricoté, le substrat ayant une première surface et une seconde surface opposée à la première surface, la seconde surface étant une surface d'interface de sang ;
un premier revêtement poreux disposé sur la première surface, le premier revêtement poreux comprenant un premier polymère ; et

un second revêtement poreux disposé sur le premier revêtement poreux, le second revêtement poreux comprenant un second polymère, et dans laquelle le premier revêtement ne pénètre pas dans la seconde surface du substrat.

**25.** Prothèse selon la revendication 24, dans laquelle la prothèse est imperméable au sang.

**26.** Prothèse selon la revendication 24, dans laquelle la prothèse a une perméabilité à l'eau inférieure à environ 0,5 ml/mn/cm$^2$ à 120 mm Hg.

**27.** Prothèse selon la revendication 24, dans laquelle le tissu est choisi dans le groupe comprenant le Dacron®, le Teflon®, le PTFE et le polyester.

**28.** Prothèse selon la revendication 24, dans laquelle le second revêtement ne pénètre que dans la première surface du substrat.

**29.** Procédé selon la revendication 24, dans lequel la prothèse comprend au moins un dispositif choisi dans le groupe comprenant une greffe de sortie d'un dispositif d'assistance vasculaire, une greffe vasculaire, une greffe de stent et un patch vasculaire.

**30.** Prothèse selon la revendication 24, dans laquelle au moins une partie de la prothèse a une forme sensiblement tubulaire.

**31.** Prothèse selon la revendication 30, dans lequel le substrat a un diamètre interne supérieur à 5 mm.

**32.** Prothèse selon la revendication 24, dans laquelle le premier polymère est au moins un polymère choisi dans le groupe comprenant un polyuréthane, une urée de polyuréthane, et un polymère de Thoralon®, et dans laquelle le second polymère est au moins un polymère choisi dans le groupe comprenant un polyuréthane, une urée de polyuréthane et un polymère de Thoralon®.

**33.** Prothèse selon la revendication 32, dans laquelle chacun parmi le premier polymère et le second polymère comprend un segment souple et un segment dur.

**34.** Prothèse selon la revendication 33, dans laquelle le segment souple a un poids moléculaire d'environ 2 000 g/mole.

**35.** Prothèse selon la revendication 33, dans lequel le segment souple comprend un ou plusieurs composés choisis dans le groupe comprenant l'oxyde de polyéthylène, l'oxyde de polypropylène, l'oxyde de polytétraméthylène, le polycarbonate, le polyoléfine, la polysiloxane, des segments souples en polyéther réalisés à partir de série homogène supérieure de diols et leurs mélanges et leurs combinaisons.

**36.** Prothèse selon la revendication 33, dans laquelle le segment dur comprend un ou plusieurs composés choisis dans le groupe comprenant le diisocyanate 4,4'-diphénylméthane, le diisocyanate de tétraméthylène, le diisocyanate d'hexaméthylène, le diisocyanate de triméthyl hexaméthylène, le diisocyanate 4,4'-dicyclohexyl méthane, le diisocyanate d'acide dimérisé, le diisocyanate d'isophorone, le diisocyanate de méthaxylène, le diisocyanate de diéthylbenzène, le 1,10-diisocyanate de décaméthylène, le 1,2-diisocyanate de cyclohexylène, le diisocyanate de 2,4-toluène, le diisocyanate de 2,6-toluène, le diisocyanate de xylène, le diisocyanate de m-phénylène, le diisocyanate, d'hexahydrotolylène (et ses isomères), le 1,5-diisocyanate de naphtylène, le 2,4-diisocyanate de 1-méthoxyphényle, le diisocyanate de 4,4'-biphénylène, le diisocyanate de 3,3-diméthoxy-4,4'-biphényle, l'éthylène diamine, les propane diamines, les butane diamines, les hexane diamines, les pentane diamines, les heptane diamines, les octane diamines, la m-xylylène diamine, les cyclohexane diamines, la 2-méthylpentaméthylène diamine, la 4,4'-méthylène dianiline, les alcanol amines et diamines, l'éthylène glycol, le diéthylène glycol, le triéthylène glycol, le 1,4-butanediol, l'alcool néopentylique, le 1,6-hexanediol, le 1,8-octanediol, les propylène glycols, le 2,3-butylène glycol, le dipropylène glycol, le dibutylène glycol, le glycérol, et des mélanges et des combinaisons de ceux-ci.

**37.** Prothèse vasculaire comprenant :

une première couche comprenant un tissu polymère ondulé tissé ou tricoté ayant une première surface ;
une deuxième couche comprenant un premier revêtement poreux disposé sur la première surface, le premier revêtement poreux comprenant un premier polymère ; et

une troisième couche comprenant un second revêtement poreux disposé sur le premier revêtement poreux, le second revêtement poreux comprenant un second polymère et dans laquelle la deuxième couche est disposée entre les première et troisième couches, et dans laquelle le premier revêtement poreux ne pénètre pas dans une seconde surface de la première couche.

38. Prothèse selon la revendication 24 ou 37, dans laquelle le second revêtement poreux a une taille de pore moyenne inférieure à 10 microns et une porosité moyenne comprise entre 50 % et 90 % par volume.

39. Prothèse selon la revendication 24 ou 37, dans laquelle la prothèse a un diamètre de vrille d'environ 12,7 mm (0,5 pouces) ou moins.

FIG. 1

**FIG. 2**

FIG. 3

FIG. 4

23

Ø .50
Ø .75
Ø 1.00
Ø 1.25
Ø 1.50
Ø 1.75
Ø 2.00
Ø 2.25
Ø 2.50
Ø 2.75
Ø 3.00

# FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5298276 A
- WO 9105522 A
- US 4604762 A
- US 4861830 A, Ward, Jr.
- US 4675361 A, Ward, Jr.
- US 5017664 A, Grasel
- US 5589563 A